(19) Europäisches Patentamt / European Patent Office / Office européen des brevets

(11) **EP 2 578 184 A1**

## (12) EUROPEAN PATENT APPLICATION

(43) Date of publication:
**10.04.2013 Bulletin 2013/15**

(51) Int Cl.:
*A61F 2/04* *(2013.01)*    *A61F 2/90* *(2013.01)*

(21) Application number: **11184326.4**

(22) Date of filing: **07.10.2011**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME** | (72) Inventors:<br>• **Schmidt, Palle Nordblad**<br>  **2800 Kgs. Lyngby (DK)**<br>• **Kubena, Petr**<br>  **500 09 Hradec Kralove (CZ)** |
| (71) Applicants:<br>• **Hvidovre Hospital**<br>  **2650 Hvidovre (DK)**<br>• **Ella-CS, s.r.o.**<br>  **506 06 Hradec Králové (CZ)** | (74) Representative: **Hauge, Sidsel et al**<br>  **Awapatent A/S**<br>  **Rigensgade 11**<br>  **1316 Copenhagen K (DK)** |

(54) **Biodegradable stent for use in the treatment of acute and chronic pancreatitis.**

(57)    The present invention relates to a transmural, self-expanding biodegradable stent (1) for the use of treating collections complicating acute and chronic pancreatitis. The stent is compressible for insertion through the gastric or duodenal wall and into the collections. The stent expands after insertion to stay in place by the specific dimensions of the stent.

Figure 1

EP 2 578 184 A1

## Description

The present invention relates to a transmural, self-expanding biodegradable stent for the use of treating collections complicating acute and chronic pancreatitis. The stent is compressible for insertion through the gastric or duodenal wall and into the collections. The stent expands after insertion to stay in place by the specific dimensions of the stent.

## Background of the invention

Acute pancreatitis (AP) is an inflammatory condition of the pancreas characterized clinically by abdominal pain and elevated levels of pancreatic enzymes in the blood. The pathogenesis is not fully understood, but a key event is activation of potent proteolytic enzymes inside the pancreas leading to autodigestive injury to the gland and its surrounding tissues. The release of pancreatic enzymes also damages the vascular endothelium resulting in microcirculatory changes, progressive ischemia, and possibly necrosis of the pancreatic and peripancreatic tissues. Finally, accumulation and activation of granulocytes and macrophages with release of inflammatory mediators may lead to worsening of the pancreatic damage. A number of conditions are known to induce AP, with gallstones and chronic alcohol abuse accounting for 75-80 percent of the cases.

About 80% of patients with AP experience a mild, self-limiting disease course with histological and functional normalization. The rest develop severe pancreatitis with varying degree of glandular destruction and possibly permanent endocrine and exocrine insufficiency. Some patients with severe AP develop systemic complications including organ dysfunction with cardiac, respiratory, and/or renal failure. Although advances in diagnostic and therapeutic interventions have led to a decrease in mortality from AP, recent large case series and literature reviews report an average mortality rate of 17% in severe AP, a rate that rises to approximately 30% in patients with infected necroses. The mortality in the first two-week period is usually due to the systemic inflammatory response syndrome and resulting organ failure and accounts for up to half of all deaths due to AP, while after two weeks it is usually due to infectious complications.

Treatment of AP is based upon the severity of the condition. Supportive care including pain management, fluid replacement, and supplemental oxygen is indicated in most patients. Nutritional support, preferably by oral or enteral route, is indicated in those patients that cannot resume normal food intake within a few days. Drainage of complicating fluid collections and surgical debridement of necrosis is indicated in case of infection, persisting pain, obstruction of the gastrointestinal or biliary tract, or leakage. Until recently, open surgery has been the most utilized treatment in such cases, but it is associated with high morbidity and mortality. It is therefore now generally accepted, that alternative minimally invasive approaches - either percutaneous or endoscopic - should be preferred.

Several reports have documented the efficacy and low complication rate of endoscopic treatment in complicated AP [References 1-9]. The treatment consists of drainage of fluid collections and debridement of pancreatic and fat necrosis inside the collections. Usually guided by endoscopic ultrasound a 15-20 mm stoma through the posterior wall of the stomach or the duodenum to the area of interest is established by needle puncture, creation of a small channel by diathermia, and finally balloon dilatation of the channel to its final diameter. Hereafter, two or more double pigtail stents and usually also a nasal flushing catheter (for flushing saline and antibiotics into the cavity) are placed through the stoma. This treatment allows the enzyme-rich pancreatic juice, pus, and necrotic debris to pass from the retroperitoneal space to the stomach lumen and further down the gastrointestinal tract, and eventually the collection to heal.

Chronic pancreatitis (CP) is a syndrome involving progressive inflammatory changes in the pancreas that result in permanent structural damage, which leads to impairment of exocrine and endocrine function. Alcohol abuse and cigarette smoking are the most common causes of CP in western countries, though in quite a few patients the disease is idiopathic. In South India and other parts of the tropics, tropical pancreatitis is the most common cause, commonly affecting children. Other less common causes include genetic and autoimmune diseases. The pathophysiology of CP is poorly understood, though as in AP intrapancreatic zymogen activation is believed to play a central role.

The two primary clinical manifestations of CP are abdominal pain and pancreatic insufficiency. Abdominal pain is a dominant feature in most patients with CP, leading to dependency of opioids in many. Patients with severe CP usually develop exocrine and endocrine insufficiency with fat malabsorption and diabetes. Chronic pancreatitis may be associated with a variety of complications. These include pseudocyst formation, bile duct or duodenal obstruction, pancreatic ascites or pleural effusion, splenic vein thrombosis, pseudoaneurysms, and pancreatic cancer.

Besides pain management and treatment of pancreatic insufficiency, endoscopic therapy remains an important part of the treatment in CP. Relief of pancreatic duct obstruction or leakage by endoscopic dilatation and stent insertion, intra- or extracorporeal crushing of intraductal stones, and drainage of fluid collections are just some of the options.

Endoscopic drainage of fluid collections (pseudocysts) associated with CP may be done either via the papilla of Vater (or the minor papilla) or transmurally, with puncture of the gastric or duodenal wall followed by insertion of one or more small-caliber plastic stents to drain the collection.

The transmural method of drainage in both AP and CP as described above is, however, associated with some drawbacks.

It has been observed that the transmural stomas - both in patients with acute and chronic pancreatitis - tend to close within a few days after their formation leading to retention of infected fluid and/or necrotic material in the drained cavity and worsening of the patient's condition. This makes it necessary to redilatate the stoma often several times during the treatment course, which is stressful for the patient, is time consuming, and increases the cost of the treatment. A few preliminary reports on implantation of self-expanding metal stents have been published [References 10-13], using either biliary, esophageal, or custom made stents. A significant potential problem with metal stents, however, is that they must be covered (by for example PTFE or silicone) to be removable and therefore may migrate into the collection. In case of migration an operation in order to remove the stent may become necessary. Another possible complication is erosion of the metal stent into major vessels in the cavity leading to life-threatening hemorrhage.

Therefore, there is a need for an improved stent that eliminates the need for repeated dilatations of the stoma, saves procedure time and costs, reduces the risk of secondary infections, and thereby is more comfortable and safe for the patients.

**Summary of the invention**

In one aspect the present invention relates to a transmural, self-expanding stent having a total length comprised of flanges forming two opposing ends and an intermediate section having a diameter. The transmural, self-expanding stent is for creating a passage that allows drainage of pancreatic fluid collections and debridement of pancreatic and/or peripancreatic necroses, caused by acute or chronic pancreatitis, from a predefined area into the gastric or duodenal lumen, wherein the transmural, self-expanding stent is made of a biodegradable material, and the ratio between the diameter of the intermediate section and the length of the intermediate section is between 0.27 to 1.25.

Thus, in the first aspect, the present invention provides a transmural, self-expanding stent which is suitable for the treatment of acute and chronic pancreatitis.

The transmural, self-expanding stent having the specified features solves the problem of spontaneous closure of the stoma by expanding the tissue within the stoma channel and thereby facilitates continuous drainage of the collection into the gastric or duodenal lumen and then further down through the gastrointestinal tract.

In one embodiment of the present invention the diameter of the intermediate section of the transmural, self-expanding stent is between 15 and 25 mm, the length of the intermediate section is between 20 and 30 mm, the diameter of the flanges is between 40 and 50 mm, and the ratio between the diameter of the intermediate section and the length of the intermediate section is between 0.5 to 1.25.

The inventors of the present invention have surprisingly found that a transmural, self-expanding stent according to the specific dimensions of this first aspect is especially useful in the treatment of so-called walled-off necroses, i.e. encapsulated collections of fluid and necrotic debris, caused by AP. A transmural, self-expanding stent having a diameter between 15 and 25 mm, thus, provides the necessary space for insertion of the various endoscopic devices needed for treatment of these complex collections and the space needed for removal of necrotic debris from the collection.

Furthermore, the inventors have found that the stoma is kept sufficiently open for the necrotic debris to be spontaneously expelled or flushed out through the stent into the gastric or duodenal lumen and thereby, further through the gastrointestinal tract, which may in some cases eliminate the need for endoscopic necrosectomy, i.e endoscopic removal of debris from the collection, and thereby possibly reduce the risk of complications, e.g. bleeding, the need for endoscopic interventions, and the length of the hospital stay.

Additionally, the inventors have observed that ingrowth of tissue into the stent according to the present invention happened within the first days after insertion into the stoma, and thus, provides a fixation of the stent, reducing the risk of stent migration which might occur spontaneously or during endoscopic maneuvers. The encapsulation by tissue ingrowth may also delay the degradation of the stent.

In a second embodiment the transmural, self-expanding stent according to the invention has a diameter of the intermediate section between 8 and 12 mm, a length of the intermediate section between 20 and 30 mm, a diameter of the flanges between 25 and 35 mm, and a ratio between the diameter of the intermediate section and the length of the intermediate section between 0.27 and 0.6.

A transmural, self-expanding stent according to these specific dimensions of the second aspect is found to be especially useful for the treatment of pseudocysts caused by either acute or chronic pancreatitis, since such fluid collections without necrotic debris do not require insertion of an endoscope or endoscopic devices into the collection.

The diameter of the intermediate section being between 8 and 12 mm is sufficient for the pseudocysts to drain into the gastric or duodenal lumen.

Furthermore, the expansion force of the stent will keep the stoma channel sufficiently open for the period needed to promote healing of the collection, avoiding the risk of retention and secondary infection.

The transmural, self-expanding stent according to the present invention may be made of any kind of polymer, wherein the backbone comprises a heteroatom-containing polymer which confers the biodegradability. Such biodegradability can, therefore, be engineered into polymers by the judicious addition of chemical linkages such as anhydride, ester, or amide bonds amongst others.

The biodegradability must be biocompatible and meet the criteria to be qualified as biomaterial, and thereby be processable, sterilizable, and capable of controlled stability or degradation in response to biological conditions.

A suitable biodegradable material of the stent according to the present invention may be selected from the group: polydioxanone; polylactic acid; 3-hydroxypropionic acid; polyesters based on polylactide, polyglycolide, polycaprolactone, and their copolymers; polyhydroxyalkanoate; and/or modified polysaccharides such as starch, cellulose, and chitosan. Other suitable biodegradable materials may be any metal alloy which corrodes gradually. Such corrosion process should, however, be proved not the harm the human body by e.g. being partly absorbed by the tissue and partly excreted through the gastrointestinal tract.

In a preferred embodiment of the present invention the transmural, self-expanding stent is manufactured from commercially available absorbable surgical suture made of polydioxanone.

Polydioxanone degrades by random hydrolysis of its ester bonds into glyoxylic acid - the primary precursor of oxalic acid and an intermediate in the conversion of glycolic acid to glycine. The stent material is partly absorbed and partly travels through the gastrointestinal tract.

In vitro studies performed at 37 °C and pH 7 have shown that the radial force of the stent is maintained for 5 weeks, hereafter it decreases to about $\frac{2}{3}$ at week 7, and to ½ of the initial value at week 9. It is also known, that the half life of polydioxanone is shorter in gastric juice at low pH. Therefore, in case of implantation into the stomach, medical inhibition of the gastric acid secretion is mandatory.

The inventors of the present invention have found that the stent according to the present invention may keep the stoma open for up to several weeks (up to 79 days), and thereby no redilatation of the stoma is necessary.

The transmural, self-expanding stent may also be prepared either fully or in part from radiopaque composite fibers.

In another embodiment of the present invention the transmural, self-expanding stent is formed from at least one piece of biodegradable material. The manner of manufacturing the stent may be chosen according to specific requirements, and may be formed either as a single integral item, or be composed by a number of pieces.

In yet another embodiment of the present invention the material the transmural, self-expanding stent is made of is braid-interlaced together to create a regular mesh. A regular mesh provides the optimal flexibility and durability of the transmural, self-expanding stent before, during and after insertion in the stoma.

Additionally, the cross points arising from the braid-interlaced mesh may be non-fused or fused together so as to allow the mesh restricted freedom of movement, preferably the cross points are non-fused.

If the cross points are fused together a more rigid structure of the transmural, self-expanding stent will be provided. This may be beneficial in certain cases where the patient's tissue demands a stent with higher expansion force to keep the stoma channel open.

In cases where a more flexible and dynamic structure of the transmural, self-expanding stent will present the improved treatment, the non-fused cross points are preferred. When not fused together the transmural, self-expanding stent is allowed an unrestricted movement and may be easier to compress prior to the insertion into the stoma.

The non-fused cross points provide a stent with an optimal degree of compression prior to insertion into the stoma. The transmural, self-expanding stent according to the invention may be compressed to fit into an insertion tube allowing insertion either through the endoscope or over the wire along the endoscope.

In another embodiment the radiopaque markers on the transmural, self-expanding stent are made of a metal, preferably gold. Other types of metal which do not corrode or in any other way harm the human body may also be used. Other metals such as platinum, iridium or blends thereof are preferred if gold is not used.

However, radiopaque markers based on degradable polymers filled with nano and/or micro particles in the amount from approximately 10 to approximately 60% based on weight are preferred. The filling material may be made from materials like $BaSO_4$, $(BiO)_2CO_3$ and compounds containing wolfram (W) or its alloys mixed with a degradable polymer of any kind. Preferably W or its alloys is mixed with the same kind of polymer the transmural, self-expanding stent is prepared of.

Radiopaque markers of metal are easily recognized by radiography, which is an important, non-invasive and useful method of controlling the placement of the transmural, self-expanding stent according to the invention.

The number of radiopaque markers is at least one, preferably three, placed at each opposing ends of the transmural, self-expanding stent.

The transmural, self-expanding stent may further comprise a string interlaced into one of the opposing ends. The string may be any kind of string that can resist the force of a pull in order to correct the position of the transmural, self-expanding stent within the stoma. The preferred kind of string is a silk thread as this is a strong material and easily is passed through the intestinal tract once the stent is degrading, and is harmless towards the human body before and during the passage through the intestinal tract.

In another embodiment according to the present invention the transmural, self-expanding stent comprises approximately at the center of the intermediate section at least one detection band.

The detection band(s) may be a color band of any color which is visible when using optical sensor means during endoscopy. Alternatively, it may be graft-like band(s) or metal band(s) which also provides the possibility of visual detection by an optical sensor means during endoscopy.

When the detection band(s) is a color band it may be made of the same biodegradable material as the transmural, self-expanding stent but in a different color. It may further be made of any kind of material approved to be used inside the human body.

The stent according to the present invention may further comprise a covering or a coating. Such covering may be any kind of membrane or expandable material which either may surround the mesh of the stent, may be attached to the inside of the mesh or may be integrated into the mesh of the stent, and thereby, expand together with the mesh upon insertion into the stoma. Once inserted into the stoma the covering may have the effect of stopping or preventing bleeding from the creation of the stoma. The covering may also prevent a potential rapid ingrowth of the tissue into the mesh of the stent and thereby extend the time where the stoma stays open.

## Description of the drawings

Figure 1 is a schematic illustration of the transmural, self-expanding biodegradable stent according to the invention.

Figure 2 is a schematic illustration of the transmural, self-expanding stent according to the invention seen as a side view.

Figure 3 is a schematic illustration of the transmural, self-expanding stent according to the invention seen from one of the opposing ends.

Figure 4 is a schematic illustration of the transmural, self-expanding stent according to the invention seen from a diagonal sectional view.

## Detailed description of the invention

When referring to the transmural, self-expanding stent according to the invention, the terms "transmural, self-expanding stent", "the stent according to the invention", and "the stent 1" may be used interchangeably.

Throughout the description and the claims the reference numerals are the same when referring to any of the drawings.

Additionally, it is contemplated that the dimensions illustrated in any of the figures should not be considered to be a limitation of the present invention.

The predefined area for which the stent of the present invention is to be inserted is determined by real-time ultrasound during the endoscopic procedure. It may be determined by appearance of an area which indicates a walled-off necrosis and/or a collection of fluid.

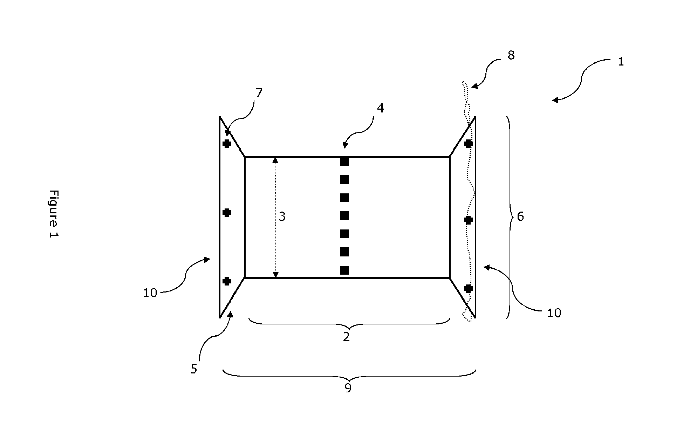

Now referring to figure 1 the invention will be described in more detail. Figure 1 illustrates an embodiment of the transmural, self-expanding stent according to the invention.

The transmural, self-expanding stent 1 comprises an intermediate section 2 having a diameter 3 and at least one detection band 4 approximately in the middle of the intermediate section 2, and one flange 5 at each opposing end 10 of the stent 1 having a diameter 6, and radiopaque markers 7.

In the embodiment shown and described, the number of radiopaque markers 8 is three at each opposing end 10 of the stent 1. Other configurations of the present invention are conceivable as well. However, the number may vary from at least one.

In the embodiment shown, one of the flanges 5 comprises a string 8.

The total length 9 of the stent 1 includes the length of the intermediate section 2 and the flanges 5 at each opposing end 10, and may be any length from 25 mm to 65 mm, preferably between 40 mm and 55 mm. In the embodiment shown, the total length 9 is 55 mm.

The length of the intermediate section 2 may be any length from 15 mm to 45 mm, preferably between 20 mm and 35 mm, most preferred 30 mm. In the embodiment shown, the length of the intermediate section 2 is 30 mm.

The diameter 3 of the intermediate section may be between 8 and 25 mm. In the embodiment shown, the diameter 3 is 25 mm.

The diameter 6 of the flanges 5 may be between 25 and 50 mm. In the embodiment shown, the diameter 6 is 45 mm.

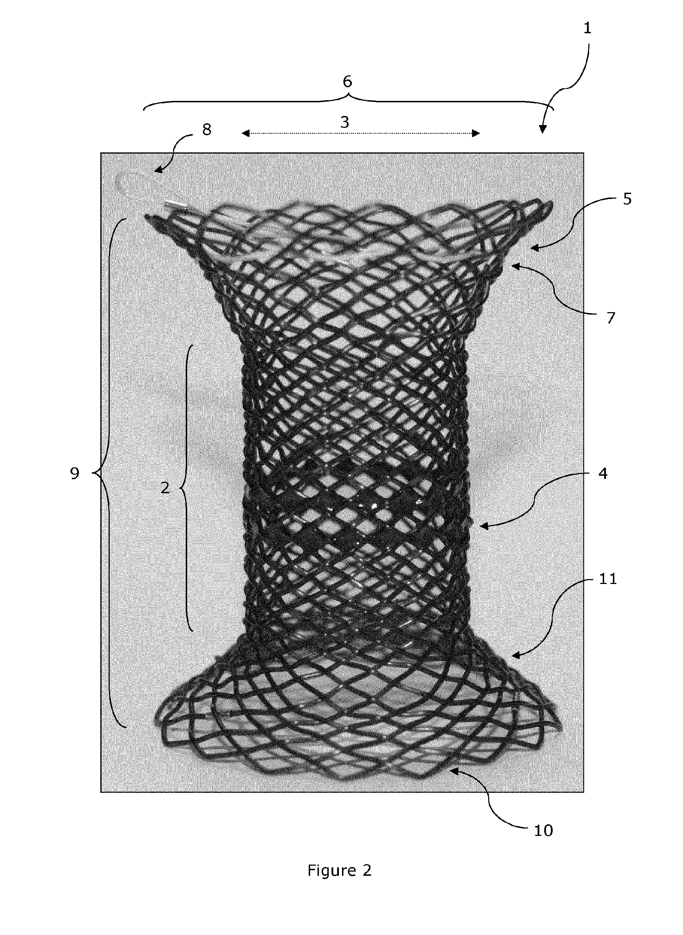

Figure 2 illustrates the same embodiment of the transmural, self-expanding stent 1 as illustrated in figure 1 according to the invention.

The transmural, self-expanding stent 1 according to this embodiment comprises an intermediate section 2 having a diameter 3 and two detection bands 4 approximately in the middle of the intermediate section 2, and one flange 5 at each opposing end 10 of the stent 1 having a diameter 6, and radiopaque markers 7. The number of radiopaque markers 7 is three at each opposing end 10 of the stent 1. One of the flanges 5 comprises a string 8.

The total length 9 of the stent 1 includes the length of the intermediate section 2 and the flanges 5 at each opposing end 10, and may be any length from 25 mm to 65 mm, preferably between 40 mm and 55 mm. In the embodiment shown, the total length 9 is 55 mm.

The length of the intermediate section 2 may be any length from 15 mm to 45 mm, preferably between 20 mm and 35 mm, most preferred 30 mm. In the embodiment shown, the length of the intermediate section 2 is 30 mm.

The transmural, self-expanding stent 1 is braided to a mesh of a left and a right winding providing cross points 11 which enables the stent 1 to be compressed prior to insertion into the stoma. Once the stent 1 is inserted it will expand to the intended expansion of the stoma. In the preferred embodiment of the present invention the cross points 11 are non-fused cross points 11.

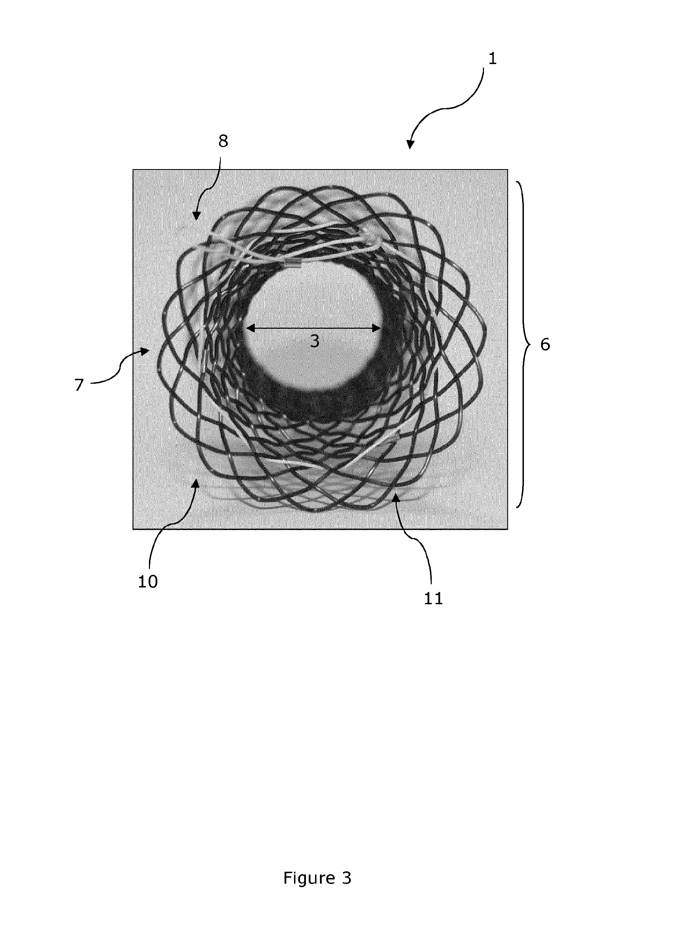

Figure 3 illustrates a sectional view of the transmural, self-expanding stent 1 according to the invention.

The transmural, self-expanding stent 1 is viewed from one of the opposing ends 10 indicating the diameter 3 of the intermediate section 2 of the stent 1. It further illustrates the diameter 6 of one of the opposing ends 10 as well as the radiopaque markers 7. The stent 1 further comprises the string 8.

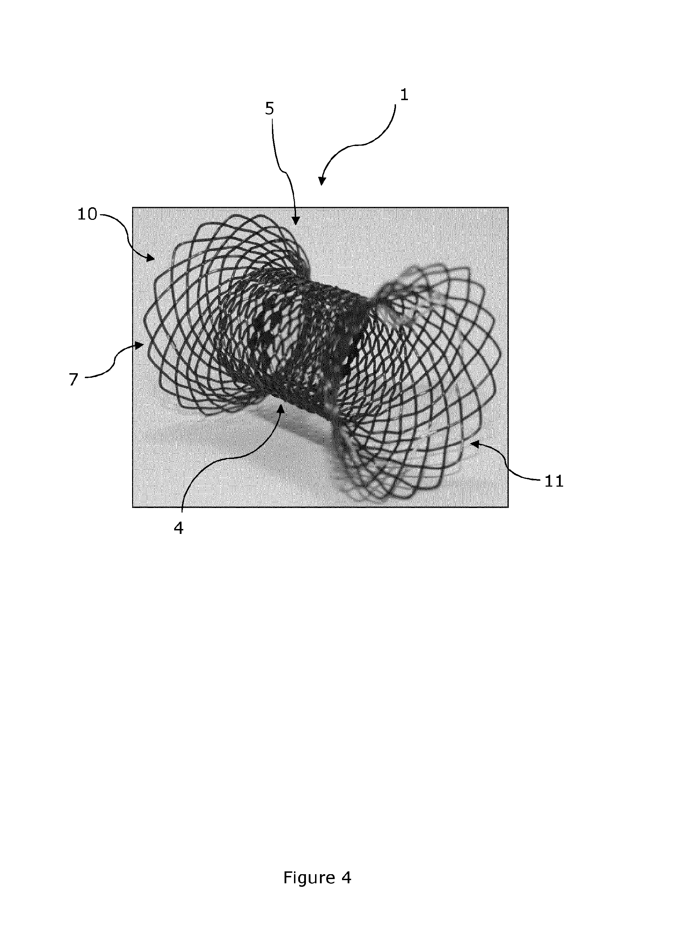

Figure 4 illustrates the transmural, self-expanding stent 1 according to the present invention in a diagonal sectional view.

In the embodiment shown, the stent 1 comprises two opposing ends 10 having a flange 5 and three radiopaque markers 7 at each opposing end 10. Furthermore, two detection bands 4 are present approximately in the middle of the intermediate section 2.

The transmural, self-expanding stent 1 is braided to a mesh of a left and a right winding providing cross points 11 which enables the stent 1 to be compressed prior to insertion into the stoma. Once the stent 1 is inserted it will expand to the intended expansion of the stoma. In the preferred embodiment of the present invention the cross points 11 are non-fused cross points 11.

**Examples**

*Example 1:*

A 41-year old, overweight (175 kg, 177 cm, Body Mass Index (BMI) 56) male was admitted with abdominal pain of 19 days duration. Contrast enhanced CT (CECT) showed necrosis of the body and tail of pancreas. Ten days after admission the patient's condition deteriorated and he was transferred to the Intensive Care Unit (ICU) due to respiratory, cardiac, and renal failure. He was treated with mechanical ventilation, temporary pacemaker because of sinoatrial block and cardiac arrest, and hemodialysis. A repeated CECT showed a 12 x 16 x 17 cm walled-off necrosis. Endoscopic ultrasound-guided, transgastric drainage was done. An 18-mm stoma was created through the posterior wall of the stomach and two 7-Fr double pigtail stents and a nasal lavage catheter was inserted. To reduce the risk of secondary infection due to retention in the cavity and the need for endoscopic interventions it was decided to replace the pigtail stents with a new, custom-made, self-expanding, biodegradable stent according to the present invention.

Follow-up endoscopy done 3 days after stent placement showed that the stent had become fixed to the rim of the stoma by an in-growth of gastric epithelium through the stent mesh. This did not affect stent patency.

For 2½ months (79 days) after implantation of the stent, a therapeutic gastroscope (Olympus GIF-1TQ160) with an outer diameter of 11.3 mm could easily pass through the stent to inspect the cavity. It was observed that necrotic debris was spontaneously rejected and could be flushed out into the stomach lumen through the stent. The patient stayed in the ICU for 42 days, during which he was on controlled ventilation for 41 days, needed hemodialysis for 11 days, and cardiac stimulation for 7 days. The long-lasting immobilization was complicated by a deep sacral pressure ulcer, which had to be treated with surgical revision and hyperbaric oxygen therapy. The patient was discharged from the hospital 157 days after admission. The first sign of stent degradation was seen 79 days after deployment. A final gastroscopy done 148 days after implantation of the stent according to the present invention showed, that the stent had completely degraded and the transgastric stoma had healed.

*Example 2:*

A 57-year old woman developed gallstone pancreatitis two months after an uneventful laparoscopic cholecystectomy. CECT showed necrosis of the neck and body of pancreas with signs of a disconnected pancreatic tail syndrome. Two and a half months after debut of the pancreatitis, the patient had developed a 10 x 10 x 20 cm walled-off necrosis and splenic vein thrombosis with several perigastric collaterals. She was constantly symptomatic with abdominal pain and nausea and a low-grade fever around 38°C. She was then referred for transgastric drainage.

With great difficulty a small, vessel-free area suitable for drainage was localized. The stoma was dilated to a final diameter of only 13 mm to avoid bleeding after which the cavity was emptied for 1.5 L of non-infected pancreatic fluid. Inspection of the cavity showed large amounts of necrotic debris. To reduce the risk of secondary infection and the need for endoscopic interventions it was decided to insert a new, custom-made, self-expanding, biodegradable stent according to the present invention. After drainage the patient rapidly became symptom-free and could be discharged from hospital. A follow-up gastroscopy 8 days after implantation showed fixation of the stent by epithelial in-growth, which did not affect stent patency. To accelerate healing endoscopic necrosectomy was done once a week for four weeks, mostly on outpatient basis, after which the cavity had healed. To follow the process of stent degradation the patient was scheduled for repeated gastroscopy every 2-4 weeks. The first sign of stent degradation was observed 54 days after implantation, and complete degradation with closure of the stoma was observed after 105 days.

*Example 3:*

A 79-year old man with known prostate cancer was admitted with common bile duct stones and cholangitis. After Endoscopic Retrograde CholangioPancreatography (ERCP) with biliary sphincterotomy and stone extraction he developed acute pancreatitis complicated by an 11 x 11 x 21 cm walled-off, peripancreatic necrosis. At the referring hospital he had a laparotomy because of abdominal compartment syndrome, but the necrotic collection was not drained during the operation. Because of the large collection it was impossible to close his abdomen, and a VAC abdominal dressing was applied. With open abdomen and severe diarrhea due to pseudomembranous enterocolitis with *Clostridium difficile* ribotype 027 caused by long-lasting treatment with broad spectrum antibiotics the patient was referred for endoscopic drainage.

CECT showed the collection to be divided into two major collections connected by a narrow channel. The upper collection was located behind the stomach and the lower was located caudal to the 3$^{rd}$ part of duodenum. To access the upper collection a 20-mm stoma was created through the posterior stomach wall. To reduce the need of endoscopic interventions it was decided to insert a new, custom-made, self-expanding, biodegradable stent according to the present invention. Inspection of the cavity immediately after stent deployment showed large amounts of necrotic fatty tissue, most of which was firmly adherent. A naso-cystic lavage catheter was inserted through the stent according to the present invention to flush the cavity while waiting for the necrotic tissue to loosen, and the lower collection was treated by transcutaneous drainage. Every 1-2 weeks the patient was brought back for gastroscopy with removal of loosely adherent necroses. By injecting saline mixed with indigo carmine through the external drain in the lower collection during endoscopy it was confirmed that the two collections were connected, since indigo carmine colored pus showed up behind necrotic debris in the bottom of the drained (upper) collection. Further, a fistula between the lower collection and the transverse colon was visualized by contrast injection. After nearly two months of endoscopic treatment most of the necrotic debris had been removed from the upper cavity, but the patient continued to show symptoms of infection. A final necrosectomy done in order to access the lower collection was complicated by a severe arterial bleeding. Endoscopic treatment with injection of epinephrine and clipping was unsuccessful, and a 20-mm dilation balloon was instead inserted through the stent according to the present invention to successfully seal the cavity. An emergency CT-angiography showed a branch of the gastro-duodenal artery to be the most probable bleeding source, and embolization was performed with lasting hemostasis. Despite this the patient died from sepsis a few days later. A final gastroscopy done 68 days after the index endoscopy showed the stent according to the present invention well placed and fully patent with no signs of degradation.

In all experiments proper drainage at all time points was observed. Improved access to the collections, facilitated necrosectomy, and accelerated healing were observed as well. Endoscopy through the stent was possible in all cases without redilatation of the stoma for up to 79 days after stent deployment. There were no cases of stent migration because of ingrowth of gastric epithelium through the stent mesh. Incipient and complete degradation of the stent was observed in two of the patients 54-79 days and 104-148 days after deployment, respectively. In the third patient, who unfortunately died because of septic complications to his disease, no sign of degradation was observed 68 days after stent deployment. Stent delivery was easy in all cases.

**References**

1. Baron TH, Thaggard WG, Morgan DE et al. Endoscopic therapy for organized pancreatic necrosis. Gastroenterology 1996;111:755-64.

2. Seifert H, Wehrmann T, Schmitt T et al. Retroperitoneal endoscopic debridement for infected peripancreatic necrosis. Lancet. 2000 Aug 19;356(9230): 653-5.

3. Baron TH, Harewood GC, Morgan DE et al. Outcome differences after endoscopic drainage of pancreatic necrosis, acute pancreatic pseudocysts, and chronic pancreatic pseudocysts. Gastrointest Endosc 2002;56:7-17.

4. Seewald S, Groth S, Omar S et al. Aggressive endoscopic therapy for pancreatic necrosis and pancreatic abscess: a new safe and effective treatment algorithm (videos). Gastrointest Endosc 2005;62: 92-100.

5. Voermans RP, Veldkamp MC, Rauws EA et al. Endoscopic transmural debridement of symptomatic organized pancreatic necrosis (with videos). Gastrointest Endosc 2007;66:909-16.

6. Escourrou J, Shehab H, Buscail L et al. Peroral transgastric/transduodenal necrosectomy: success in the treatment of infected pancreatic necrosis. Ann Surg 2008;248: 1074-80.

7. Seifert H, Biermer M, Schmitt W et al. Transluminal endoscopic necrosectomy after acute pancreatitis: a multicentre study with long-term follow-up (the GEPARD Study). Gut 2009;58:1260-6.

8. Ross A, Gluck M, Irani S et al. Combined endoscopic and percutaneous drainage of organized pancreatic necrosis. Gastrointest Endosc 2010;71: 79-84.

9. Gardner TB, Coelho-Prabhu N, Gordon SR et al. Direct endoscopic necrosectomy for the treatment of walled-off pancreatic necrosis: results from a multicenter U.S. series. Gastrointest Endosc 2011;73: 718-26.

10. Antillon MR, Bechtold ML, Bartalos CR et al. Transgastric endoscopic necrosectomy with temporary metallic esophageal stent placement for the treatment of infected pancreatic necrosis (with video). Gastrointest Endosc 2009;69:178-80.

11. Belle S, Collet P, Post S et al. Temporary cystogastrostomy with self-expanding metallic stents for pancreatic necrosis. Endoscopy 2010;42: 493-495.

12. Kim J, Shah JN, Marson F et al. Endoscopic drainage of pancreatic fluid collection with covered self-expanding metal stents under EUS guidance. Abstract T1459, DDW 2010.

13. Penn DE, Draganov PE, Wagh MS et al. The use of fully-covered self-expanding metal stents for endoscopic ultrasound guided drainage of pancreatic pseudocysts. Abstract SU 1404, DDW 2011.

**Claims**

1. A transmural, self-expanding stent (1) having a total length (9) comprised of flanges (5) forming two opposing ends (10) having a diameter (6), and an intermediate section (2) having a diameter (3), the transmural stent (1) creating a transgastric or transduodenal passage for draining fluid collections and performing debridement of pancreatic and/or peripancreatic necroses, caused by acute or chronic pancreatitis, from a predefined area into the gastric or duodenal lumen **characterized in that** the transmural, self-expanding stent (1) is made of a biodegradable material, the ratio between the diameter (3) of the intermediate section (2) and the length of the intermediate section (2) is ranging from 0.27 to 1.25.

2. The transmural, self-expanding stent (1) according to claim 1, wherein the diameter (3) of the intermediate section (2) is between 15 and 25 mm, the length of the intermediate section (2) is between 20 and 30 mm, the ratio between the diameter (3) and the length of the intermediate section (2) is from 0.5 to 1.25, and the diameter (6) of the flanges (5) is between 40 and 50 mm, for use in the treatment of walled-off necroses caused by acute pancreatitis.

3. The transmural, self-expanding stent (1) according to claim 1, wherein the diameter (3) of the intermediate section (2) is between 8 and 12 mm, the length of the intermediate section (2) is between 20 and 30 mm, the ratio between the diameter (3) and the length of the intermediate section (2) is from 0.27 to 0.60, and the diameter (6) of the flanges (5) is between 25 and 35 mm for use in the treatment of pseudocysts caused by acute or chronic pancreatitis.

4. The transmural, self-expanding stent (1) according to any of the preceding claims, wherein the biodegradable material is selected from the group: polydioxanone; polylactic acid; 3-hydroxypropionic acid; polyesters based on polylactide, polyglycolide, polycaprolactone, and there copolymers; polyhydroxyalkanoate; modified polysaccharides such as starch, cellulose, and chitosan, and/or any kind of metal alloy, allowing the stent to be gradually degraded.

5. The transmural, self-expanding stent (1) according to any of the preceding claims, wherein the biodegradable material is polydioxanone.

6. The transmural, self-expanding stent (1) according to any of the preceding claims, formed from at least one piece of biodegradable material.

7. The transmural, self-expanding stent (1) according to any of the preceding claims, wherein the material is braid-interlaced together to create a regular mesh.

8. The transmural, self-expanding stent (1) according to any of the preceding claims, wherein the cross points (11) may be non-fused or fused together as to allow the mesh restricted freedom of movement, preferably the cross points (11) are non-fused.

9. The transmural, self-expanding stent (1) according to any of the preceding claims, wherein the radiopaque markers (7) are made of a metal, preferably gold.

10. The transmural, self-expanding stent (1) according to any of the preceding claims, wherein the number of radiopaque markers (7) is at least one, preferably three, in each opposing ends of the self-expanding biodegradable stent (1).

11. The transmural, self-expanding stent (1) according to any of the preceding claims, wherein the thread (8) is a silk thread.

12. The transmural, self-expanding stent (1) according to any of the preceding claims, wherein the centre of the intermediate section is marked by at least one detection band (4) which may be color band(s), graft-like band(s), or metal band(s).

Figure 1

Figure 2

Figure 3

Figure 4

Europäisches
Patentamt

European
Patent Office

Office européen
des brevets

# EUROPEAN SEARCH REPORT

Application Number

EP 11 18 4326

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/161341 A1 (STINSON JONATHAN S [US] ET AL) 31 October 2002 (2002-10-31) * paragraphs [0037], [0041]; figure 2 * ----- | 1-19 | INV. A61F2/04 A61F2/90 |

TECHNICAL FIELDS SEARCHED (IPC)

A61F
A61M
A61B
A61J

The present search report has been drawn up for all claims

| Place of search | Date of completion of the search | Examiner |
|---|---|---|
| Munich | 8 December 2011 | Skorovs, Peteris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 18 4326

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-2011

| Patent document cited in search report | | Publication date | Patent family member(s) | | Publication date |
|---|---|---|---|---|---|
| US 2002161341 | A1 | 31-10-2002 | US 2002161341 A1 | | 31-10-2002 |
| | | | US 2004098105 A1 | | 20-05-2004 |
| | | | WO 02087469 A2 | | 07-11-2002 |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BARON TH ; THAGGARD WG ; MORGAN DE et al.** Endoscopic therapy for organized pancreatic necrosis. *Gastroenterology,* 1996, vol. 111, 755-64
- **SEIFERT H ; WEHRMANN T ; SCHMITT T et al.** Retroperitoneal endoscopic debridement for infected peripancreatic necrosis. *Lancet,* 19 August 2000, vol. 356 (9230), 653-5
- **BARON TH ; HAREWOOD GC ; MORGAN DE et al.** Outcome differences after endoscopic drainage of pancreatic necrosis, acute pancreatic pseudocysts, and chronic pancreatic pseudocysts. *Gastrointest Endosc,* 2002, vol. 56, 7-17
- **SEEWALD S ; GROTH S ; OMAR S et al.** Aggressive endoscopic therapy for pancreatic necrosis and pancreatic abscess: a new safe and effective treatment algorithm (videos. *Gastrointest Endosc,* 2005, vol. 62, 92-100
- **VOERMANS RP ; VELDKAMP MC ; RAUWS EA et al.** Endoscopic transmural debridement of symptomatic organized pancreatic necrosis (with videos. *Gastrointest Endosc,* 2007, vol. 66, 909-16
- **ESCOURROU J ; SHEHAB H ; BUSCAIL L et al.** Peroral transgastric/transduodenal necrosectomy: success in the treatment of infected pancreatic necrosis. *Ann Surg,* 2008, vol. 248, 1074-80
- **SEIFERT H ; BIERMER M ; SCHMITT W et al.** Transluminal endoscopic necrosectomy after acute pancreatitis: a multicentre study with long-term follow-up (the GEPARD Study. *Gut,* 2009, vol. 58, 1260-6

- **ROSS A ; GLUCK M ; IRANI S et al.** Combined endoscopic and percutaneous drainage of organized pancreatic necrosis. *Gastrointest Endosc,* 2010, vol. 71, 79-84
- **GARDNER TB ; COELHO-PRABHU N ; GORDON SR et al.** Direct endoscopic necrosectomy for the treatment of walled-off pancreatic necrosis: results from a multicenter U.S. series. *Gastrointest Endosc,* 2011, vol. 73, 718-26
- **ANTILLON MR ; BECHTOLD ML ; BARTALOS CR et al.** Transgastric endoscopic necrosectomy with temporary metallic esophageal stent placement for the treatment of infected pancreatic necrosis (with video. *Gastrointest Endosc,* 2009, vol. 69, 178-80
- **BELLE S ; COLLET P ; POST S et al.** Temporary cystogastrostomy with self-expanding metallic stents for pancreatic necrosis. *Endoscopy,* 2010, vol. 42, 493-495
- **KIM J ; SHAH JN ; MARSON F et al.** *Endoscopic drainage of pancreatic fluid collection with covered self-expanding metal stents under EUS guidance,* 2010
- **PENN DE ; DRAGANOV P ; WAGH MS et al.** *The use of fully-covered self-expanding metal stents for endoscopic ultrasound guided drainage of pancreatic pseudocysts,* 2011